# EUROPEAN PATENT APPLICATION

(11) **EP 4 461 252 A1**
(43) Date of publication of application: **13.11.2024**
(21) Application number: 24175177.5
(22) Date of filing: 10.05.2024
(51) Int. Cl.: A61B 90/00, A61B 34/00, A61B 1/00, A61B 34/10, A61B 34/20, A61B 90/30

(54) **SYSTEMS AND METHODS FOR TRACKING LOCATIONS OF INTEREST IN ENDOSCOPIC IMAGING**

(30) Priority: 10.05.2023 US 202363501391 P
(71) Applicant: Stryker Corporation, Kalamazoo, MI 49002 (US)
(72) Inventor: HUNTER, Cole Kincaid, Kalamazoo, 49002 (US); FOUTS, Brian, Kalamazoo, 49002 (US); ZEH, Christopher, Kalamazoo, 49002 (US)
(74) Representative: V.O.

(57) **Abstract**

A system for tracking a location of interest of anatomy of a patient in medical imaging includes, a computing system configured for receiving a series of medical imaging video frames captured by a medical imager imaging the anatomy of the patient; analyzing at least one frame of the series of medical imaging video frames to determine a position of the location of interest relative to the medical imager; determining at least one estimate of relative motion between the medical imager and the anatomy of the patient; and tracking the position of the location of interest relative to the medical imager based on the position of the location of interest determined from the at least one frame and the at least one estimate of relative motion between the medical imager and the anatomy of the patient.

## Description

### CROSS-REFERENCE TO RELATED APPLICATIONS

This application claims the benefit of U.S. Provisional Application No. 63/501,391, filed May 10, 2023, the entire contents of which are hereby incorporated by reference herein.

### FIELD

This disclosure generally relates to endoscopic imaging and, more specifically, to determining and/or tracking one or more locations of interest in endoscopic imaging.

### BACKGROUND

Minimally invasive surgery generally involves the use of a high-definition camera coupled to an endoscope inserted into a patient to provide a surgeon with a clear and precise view within the body. In many surgical contexts, the surgeon is often required to make precise distance measurements within the surgical space so as to accurately perform a given procedure during the surgery. Prior methods of estimating measurements in a surgical space can include the use of hooked probes in the surgical cavity; however, this method can be inaccurate and can be limited by the trajectory that the hooked probe can be positioned within the cavity. Other prior methods include connecting a piece of suture to an anchor, marking the suture with a sterile marker outside of the joint, drawing the suture out across the anatomy, and marking the suture again. This method, while perhaps producing an accurate result, can be complex and time consuming for a surgeon to undertake during a surgical procedure.

Conventionally, the surgeon may estimate distances by viewing video data supplied by an endoscopic device. Using the video data, as well as the surgeon's general knowledge of the anatomy in the surgical space, the surgeon can estimate distances between two points in the surgical space. However, this "human" method of estimating distances can be entirely reliant on the surgeon's ability to estimate distances based on the images provided by the endoscopic device. Surgeons can improve the accuracy of their measurements by placing measuring devices in the surgical space (such as a ruler) to measure the distance between two points. However, doing so requires the surgeon to place additional objects in the surgical space which is limited by the surgical portal location and trajectory.

### SUMMARY

According to an aspect, systems and methods track locations of interest of anatomy of a patient in endoscopic imaging by estimating the motion of an endoscopic imager relative to the anatomy. The three-dimensional position of a location of interest can be determined from an endoscopic image that captures a surgical tool that has a fiducial marker and is used to indicate the location of interest in the image. The motion of the endoscopic imager over time can be estimated to track the position of the location of interest relative to the camera as the camera moves relative to the anatomy.

Motion of the endoscopic imager can be estimated using one or more sensors to monitor the motion of the endoscopic imager, such as an inertial measurement unit positionally fixed relative to the endoscopic imager or a camera-based tracking system that monitors motion of a tracker mounted to the endoscopic imager. Alternatively or additionally, an image analysis method can be used to estimate the relative motion of the endoscopic imager. Different endoscopic imager motion estimation methods can be used together for tracking the position of the location of interest over time. The tracked position can be used to update a visualization with a graphical indication of the location of interest and/or to generate a measurement between two tracked locations of interest.

According to an aspect a system for tracking a location of interest of anatomy of a patient in medical imaging comprises a computing system configured for: receiving a series of medical imaging video frames captured by a medical imager imaging the anatomy of the patient; analyzing at least one frame of the series of medical imaging video frames to determine a position of the location of interest relative to the medical imager; determining at least one estimate of relative motion between the medical imager and the anatomy of the patient; and tracking the position of the location of interest relative to the medical imager based on the position of the location of interest determined from the at least one frame and the at least one estimate of relative motion between the medical imager and the anatomy of the patient.

Optionally, the computing system is configured for receiving medical imager motion data associated with motion of the medical imager and determining the at least one estimate of relative motion between the medical imager and the anatomy of the patient based on the medical imager motion data.

Optionally, the system comprises a motion sensor system configured to be mounted to the medical imager. The medical imager motion data can then comprise data from the motion sensor system.

Optionally, the medical imager is an endoscopic imager and the motion sensor system is mounted to an endoscope of the endoscopic imager. The motion sensor system can be mounted to a light post of the endoscope. The motion sensor system can be configured to generate electrical energy from light directed through the light post. The motion sensor system can be configured to transmit the data wirelessly, such as to the computing system. Optionally, the system is configured for calibrating the motion sensor system at least once during a medical imaging session.

Optionally, the computing system is configured for determining the at least one estimate of relative motion between the medical imager and the anatomy of the patient based on an offset between a location of the motion sensor system and a predetermined location on the medical imager. The computing system can be configured for receiving a user input associated with selection of the offset. The computing system can be configured for determining the offset by imaging an optical calibration object by the medical imager.

Optionally, the system comprises at least one sensor that is spaced apart from the medical imager, and wherein the medical imager motion data comprises data from the least one sensor. The at least one sensor that is spaced apart from the medical imager can comprise a camera that captures images of at least one tracking object associated with the medical imager.

Optionally, the computing system is configured for determining the at least one estimate of relative motion between the medical imager and the anatomy of the patient by analyzing a plurality of frames of the series of medical imaging video frames. The computing system can be configured for analyzing a plurality of frames of the series of medical imaging video frames using a machine learning model to identify matched key points in the plurality of frames and determining the at least one estimate of relative motion between the medical imager and the anatomy of the patient based on differences in location of the matched key points. Analyzing the plurality of frames of the series of medical imaging video frames can comprise determining at least one pixel motion vector.

Optionally, the computing system is configured for analyzing the at least one frame of the series of medical imaging video frames to determine the position of a location of interest by locating the location of interest in the at least one frame based on a position of at least a portion of a tool in the at least one frame. The at least a portion of the tool can comprise a tip of the tool and the location of interest can be a location of anatomy in the at least one frame that corresponds to the tip of the tool in the at least one frame.

Optionally, the location of interest is a first location of interest, and the computing system is configured for: determining a position of a second location of interest of the anatomy of the patient relative to the medical imager based on the series of medical imaging video frames; and determining a distance between the second location of interest and the first location of interest based on a tracked position of the first location of interest relative to the medical imager and the position of the second location of interest relative to the medical imager.

Optionally, the at least one estimate of relative motion between the medical imager and the anatomy of the patient is determined using a first motion estimate at a rate of every M frames, wherein M is greater than one. Optionally, the at least one estimate of relative motion between the medical imager and the anatomy of the patient is determined using a second motion estimate at a rate of every N frames, wherein N is less than M. The first motion estimate can be a different motion estimate method than the second motion estimate. The first motion estimate can be an image analysis-based motion estimate. The second motion estimate can be a sensor-based (e.g., IMU or camera-based tracking) motion estimate. Thus, the image analysis-based motion estimate method, which may be computationally intensive, may be used less frequently than a sensor-based (e.g., IMU or camera-based tracking) motion estimate method.

Optionally, the computing system is configured for displaying a visualization comprising a graphical indication of the location of interest in association with the medical imaging video frames. The computing system can be configured for computing a measurement based on the tracked position of the location of interest and displaying the measurement in the visualization.

Optionally, the computing system is configured for determining the at least one estimate of relative motion between the medical imager and the anatomy of the patient by combining data from different medical imager motion tracking algorithms. The data from the different medical imager motion tracking algorithms can be combined using a Kalman filter.

According to an aspect, a system for tracking a location of interest of anatomy of a patient in medical imaging comprises a computing system configured for: receiving a series of medical imaging video frames captured by a medical imager imaging the anatomy of the patient; and tracking a position of a location of interest in the series of medical imaging video frames using a machine learning model that was trained with training video frames and positional data associated with a camera that captured the training video frames.

The training video frames can be synthetic video frames, and the positional data can be synthetic positional data for a simulated camera.

Optionally, the machine learning model identifies matched key points in at least two frames of the series of medical imaging video frames. The positional data can comprise data from a motion sensor system fixedly mounted relative to the camera. The positional data can comprise data from at least one sensor that is spaced apart from the camera.

Optionally, the camera is a first camera, and the at least one sensor that is spaced apart from the camera comprises a second camera that captures images of at least one tracking object associated with the first camera.

According to an aspect, a method for tracking a location of interest of anatomy of a patient in medical imaging includes, at a computing system, receiving a series of medical imaging video frames captured by a medical imager imaging the anatomy of the patient, analyzing at least one frame of the series of medical imaging video frames to determine a position of the location of interest relative to the medical imager, determining at least one estimate of relative motion between the medical imager and the anatomy of the patient, and tracking the position of the location of interest relative to the medical imager based on the position of the location of interest determined from the at least one frame and the at least one estimate of relative motion between the medical imager and the anatomy of the patient.

The method may include receiving medical imager motion data associated with motion of the medical imager and determining the at least one estimate of relative motion between the medical imager and the anatomy of the patient based on the medical imager motion data. The medical imager motion data may include data from a motion sensor system mounted to the medical imager. The medical imager may be an endoscopic imager and the motion sensor system may be mounted to an endoscope of the endoscopic imager. The endoscope can be pre-inserted prior to start of the tracking method. The motion sensor system may be mounted to a light post of the endoscope. The motion sensor system may be configured to generate electrical energy from light directed through the light post.

The motion sensor system may be configured to transmit the data wirelessly.

The method may include calibrating the motion sensor system at least once during a medical imaging session.

The at least one estimate of relative motion between the medical imager and the anatomy of the patient may be determined based on an offset between a location of the motion sensor system and a predetermined location on the medical imager. The method may include receiving a user input associated with selection of the offset. The method may include determining the offset by imaging an optical calibration object by the medical imager.

The medical imager motion data may include data from at least one sensor that is spaced apart from the medical imager. The at least one sensor that is spaced apart from the medical imager may include a camera that captures images of at least one tracking object associated with the medical imager.

The at least one estimate of relative motion between the medical imager and the anatomy of the patient may be determined by analyzing a plurality of frames of the series of medical imaging video frames. Analyzing the plurality of frames of the series of medical imaging video frames may include using a machine learning model to identify matched key points in the plurality of frames and determining the at least one estimate of relative motion between the medical imager and the anatomy of the patient based on differences in location of the matched key points. Analyzing the plurality of frames of the series of medical imaging video frames may include determining at least one pixel motion vector.

Optionally, analyzing the at least one frame of the series of medical imaging video frames to determine the position of a location of interest may include locating the location of interest in the at least one frame based on a position of at least a portion of a tool in the at least one frame. The at least a portion of the tool may include a tip of the tool and the location of interest may be a location of anatomy in the at least one frame that corresponds to the tip of the tool in the at least one frame.

The location of interest may be a first location of interest, and the method may include determining a position of a second location of interest of the anatomy of the patient relative to the medical imager based on the series of medical imaging video frames, and determining a distance between the second location of interest and the first location of interest based on a tracked position of the first location of interest relative to the medical imager and the position of the second location of interest relative to the medical imager.

The at least one estimate of relative motion between the medical imager and the anatomy of the patient may be determined using a first motion estimate at a rate of every M frames, wherein M is greater than one. The at least one estimate of relative motion between the medical imager and the anatomy of the patient may be determined using a second motion estimate at a rate of every N frames, wherein N is less than M.

The method may include displaying a visualization comprising a graphical indication of the location of interest in association with the medical imaging video frames. The method may include computing a measurement based on the tracked position of the location of interest and displaying the measurement in the visualization.

Optionally, determining the at least one estimate of relative motion between the medical imager and the anatomy of the patient includes combining data from different medical imager motion tracking algorithms. The data from the different medical imager motion tracking algorithms may be combined using a Kalman filter.

According to an aspect, a method for tracking a location of interest of anatomy of a patient in medical imaging includes, at a computing system, receiving a series of medical imaging video frames captured by a medical imager imaging the anatomy of the patient, and
tracking a position of a location of interest in the series of medical imaging video frames using a machine learning model that was trained with training video frames and positional data associated with a camera that captured the training video frames.

The training video frames may be synthetic video frames, and the positional data may be synthetic positional data for a simulated camera.

The machine learning model may identify matched key points in at least two frames of the series of medical imaging video frames.

The positional data may include data from a motion sensor system fixedly mounted relative to the camera.

The positional data may include data from at least one sensor that is spaced apart from the camera. The camera may be a first camera, and the at least one sensor that is spaced apart from the camera may include a second camera that captures images of at least one tracking object associated with the first camera.

According to an aspect, a system includes one or more processors, memory, and one or more programs stored in the memory for execution by the one or more processors to cause the system to perform any of the above methods.

According to an aspect, a computer program product comprises software code portions configured to, when executed on a programmable computer, perform the steps of one or more of the methods disclosed herein.

It will be appreciated that any of the variations, aspects, features, and options described in view of the systems apply equally to the methods, and computer program product, and vice versa. It will also be clear that any one or more of the above variations, aspects, features, and options can be combined.

### BRIEF DESCRIPTION OF THE FIGURES

The invention will now be described, by way of example only, with reference to the accompanying drawings, in which:
FIG. 1A illustrates an exemplary endoscopic imaging system;
FIG. 1B illustrates an example of the mounting of an IMU package to an exemplary endoscope;
FIG. 1C is an exemplary functional block diagram of an IMU package mountable to an endoscopic imager;
FIG. 1D illustrates an example of a variation of the system of FIG. 1A in which an exemplary IMU package includes one or more light sources configured to generate the light for illuminating a surgical cavity;
FIG. 1E illustrates an example of an IMU package connected to a light post of an exemplary endoscope;
FIG. 1F is an exemplary functional block diagram of an IMU package that includes one or more light sources;
FIG. 2 is a flow diagram of an exemplary method for tracking a location of interest of anatomy of a patient in endoscopic imaging;
FIG. 3 is a flow diagram of an exemplary method for analyzing least one image of a series of endoscopic images to determine a position of a location of interest of anatomy of a patient relative to an endoscopic imager;
FIG. 4 is a flow diagram of an exemplary method for estimating relative motion of the endoscopic imager relative to the anatomy of the patient based on processing pairs of images using visual odometry;
FIG. 5 is a flow diagram of an exemplary method of using a machine learning model to generate a set of matched key points for a pair of images;
FIG. 6 illustrates an exemplary method for training a machine learning model usable for the method of FIG. 5;
FIG. 7 illustrates an exemplary method for using optical flow to estimate motion of an endoscopic imager relative to anatomy of the patient from pairs of images;
FIG. 8 illustrates an exemplary visualization of anatomy of interest in which one or more graphical indications of one or more locations of interest are relocated in the visualization based on the movement of the endoscopic imager such that the graphical indication(s) continues to accurately indicate the position of the location(s) of interest relative to the displayed scene;
FIG. 9A illustrates exemplary timing for generating an estimate of the relative motion between the endoscopic imager and the anatomy of the patient using an image-based camera motion estimation method and using that estimate to update a position of a location of interest in a displayed visualization;
FIG. 9B illustrates using an image-based camera motion estimation method less often than an IMU-based motion estimation method for tracking the position of a location of interest;
FIG. 10 illustrates an exemplary computing system;
FIG. 11A illustrates an example of an endoscopic image having an endoscope rotational orientation indicator; and
FIG. 11B illustrates an example of an endoscopic image in which the field of view portion of the endoscopic image is shifted upward.

### DETAILED DESCRIPTION

Reference will now be made in detail to implementations and examples of various aspects and variations of systems and methods described herein. Although several exemplary variations of the systems and methods are described herein, other variations of the systems and methods may include aspects of the systems and methods described herein combined in any suitable manner having combinations of all or some of the aspects described.

Described herein are systems and methods for determining the position of a location of interest of anatomy of a patient in endoscopic imaging and tracking the position of the location of interest over time by estimating the motion of an endoscopic imager. A location of interest may be indicated by a user in an endoscopic image using a surgical tool, such as a pointer tool. An image processing system may analyze an endoscopic image that captures to the location of interest and the surgical tool and may determine the position of the location of interest in three-dimensional space based on the position and orientation of a fiducial marker affixed the surgical tool and captured in the endoscopic image. The image processing system may track the position of the location of interest over time by estimating motion of the endoscopic imager and updating the position of the location of interest accordingly. The tracked position may be used to accurately display a graphical indication of the location of interest relative to displayed endoscopic images and/or may be used to measure a distance between two locations of interest indicated by the user at different times.

Different methods can be used to estimate the motion of the endoscopic imager over time, including sensor-based methods that monitor the motion of the endoscopic imager using one or more sensors and image analysis-based methods that analyze images generated by the endoscopic imager to estimate motion of the endoscopic imager based on motion of the imaged scene. Sensor-based methods may include monitoring motion of the endoscopic imager using, for example, an inertial measurement unit rigidly fixed relative to the endoscopic imager. Motion data from the inertial measurement unit can be integrated over time to estimate motion of the endoscopic imager. Sensor-based methods may include using a camera-based tracking system to track the movement of a tracker rigidly fixed to the endoscopic imager.

Image analysis-based methods for estimating endoscopic imager motion can include generating sets of matched key points between pairs of images and estimating a motion transform based on the differences in position between the pairs of images. This can include using a machine learning model trained using training images and endoscopic imager positional information generated using any of the sensor-based endoscopic imager tracking methods described herein.

Multiple different endoscopic imager motion estimating methods can be used to estimate the motion of the endoscopic imager. For example, a more accurate but more computationally intensive method, such as a machine-learning model-based image analysis method, may be used less frequently, and less computationally intensive methods, such as a sensor-based method, may be used more frequently. Endoscopic imager motion estimates from different methods can be combined, e.g. using a filter, such as a Kalman filter.

In the following description of the various examples, it is to be understood that the singular forms "a," "an," and "the" used in the following description are intended to include the plural forms as well, unless the context clearly indicates otherwise. It is also to be understood that the term "and/or" as used herein refers to and encompasses any and all possible combinations of one or more of the associated listed items. It is further to be understood that the terms "includes, "including," "comprises," and/or "comprising," when used herein, specify the presence of stated features, integers, steps, operations, elements, components, and/or units but do not preclude the presence or addition of one or more other features, integers, steps, operations, elements, components, units, and/or groups thereof.

Certain aspects of the present disclosure include process steps and instructions described herein in the form of an algorithm. It should be noted that the process steps and instructions of the present disclosure could be embodied in software, firmware, or hardware and, when embodied in software, could be downloaded to reside on and be operated from different platforms used by a variety of operating systems. Unless specifically stated otherwise as apparent from the following discussion, it is appreciated that, throughout the description, discussions utilizing terms such as "processing," "computing," "calculating," "determining," "displaying," "generating" or the like, refer to the action and processes of a computer system, or similar electronic computing device, that manipulates and transforms data represented as physical (electronic) quantities within the computer system memories or registers or other such information storage, transmission, or display devices.

The present disclosure in some examples also relates to a device for performing the operations herein. This device may be specially constructed for the required purposes, or it may comprise a general purpose computer selectively activated or reconfigured by a computer program stored in the computer. Such a computer program may be stored in a non-transitory, computer readable storage medium, such as, but not limited to, any type of disk, including floppy disks, USB flash drives, external hard drives, optical disks, CD-ROMs, magnetic-optical disks, read-only memories (ROMs), random access memories (RAMs), EPROMs, EEPROMs, magnetic or optical cards, application specific integrated circuits (ASICs), or any type of media suitable for storing electronic instructions, and each connected to a computer system bus. Furthermore, the computing systems referred to in the specification may include a single processor or may be architectures employing multiple processor designs, such as for performing distinct functions or for increased computing capability. Suitable processors include central processing units (CPUs), graphical processing units (GPUs), field programmable gate arrays (FPGAs), and ASICs.

The methods, devices, and systems described herein are not inherently related to any particular computer or other apparatus. Various general-purpose systems may also be used with programs in accordance with the teachings herein, or it may prove convenient to construct a more specialized apparatus to perform the required method steps. The required structure for a variety of these systems will appear from the description below. In addition, the present invention is not described with reference to any particular programming language. It will be appreciated that a variety of programming languages may be used to implement the teachings of the present disclosure as described herein.

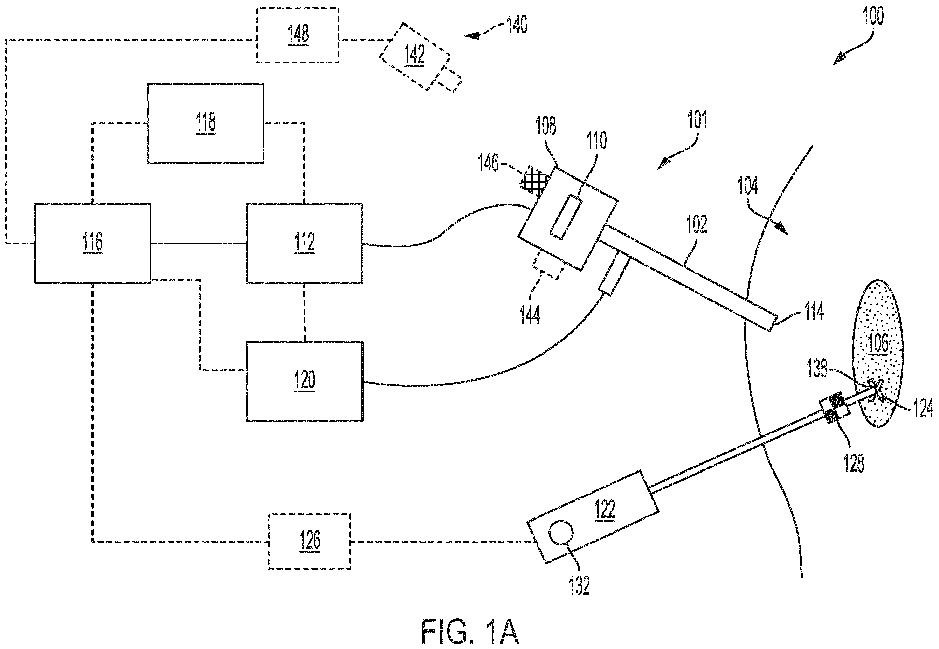

FIG. 1A illustrates an exemplary endoscopic imaging system 100. System 100 includes an endoscopic imager 101 that can include a camera head 108 mounted to an endoscope 102. As is well-known in the art, the endoscope 102 can be configured for insertion into a surgical cavity 104 for imaging tissue 106 within the surgical cavity 104 during a medical procedure. The endoscope 102 can have been pre-inserted into the surgical cavity prior to imaging and/or tracking. Light generated by a light source 120 may be directed through the endoscope 102 to the surgical cavity 104. Light reflected by and/or emitted from the tissue 106 (such as fluorescence light emitted from fluorescing targets that are excited by fluorescence excitation light provided by the light source 120) is received at the distal end 114 of the endoscope 102. The light is propagated by the endoscope 102, such as via one or more optical components (for example, one or more lenses, prisms, light pipes, or other optical components), to the camera head 108, where the light is directed onto one or more imaging sensors 110. One or more filters (not shown) may be included in the endoscope 102, in a coupler (not shown) connecting the endoscope 102 to the camera head 108, and/or in the camera head 108 for filtering a portion of the light received from the tissue 106 (such as fluorescence excitation light used for fluorescence imaging).

The one or more imaging sensors 110 generate pixel data that can be transmitted to a camera control unit 112 that is communicatively connected to the camera head 108. The camera control unit 112 can generate endoscopic images from the pixel data that show the tissue within the field of view of the endoscopic imager 101. As used herein, the term "endoscopic image(s)" encompasses single snapshot images and a sequence of video frames. As such, the terms "image," "frame," and "video frame" are used interchangeably. The endoscopic images can be transmitted to an image processing system 116 for further image processing, storage, display, and/or routing to an external device (not shown). The endoscopic images can be transmitted to one or more displays 118 from the camera control unit 112 and/or the image processing system 116 for display to medical personnel, such as for display to a surgeon for visualizing the surgical cavity 104 during a surgical procedure on a patient. The camera control unit 112 and/or the image processing system 116 may be configured to send control signals to the light source 120 and/or the camera head 108 to control one or more aspects of the imaging, such as a timing sequence of light provided by the light source 120 (e.g., a sequence of white light and fluorescence excitation light), an amount of light provided by the light source 120, and/or a gain of the one or more imaging sensors 110.

System 100 is configured to determine and/or track one or more locations of interest of the tissue 106 based at least in part on the endoscopic images generated using the endoscopic imager 101. The visual guidance can include, for example, a measurement between locations of interest of the tissue 106, a location of a feature of interest of the tissue 106, and/or a location of a hole drilled or to be drilled in the tissue 106. System 100 can determine and/or track one or more locations of interest of the tissue 106 based, at least in part, on at least one fiducial marker 128 that can be inserted into the surgical cavity 104 by a user. The fiducial marker 128 can have been pre-inserted into the surgical cavity 104 prior to start of determining and/or tracking the one or more locations of interest. The fiducial marker 128 can be used to scale features captured in one or more endoscopic images, so that the system can determine the three-dimensional positions of one or more locations of interest of the tissue 106 based on where the one or more locations of interest appear in the endoscopic image.

System 100 can include a surgical tool 122 that can include the at least one fiducial marker 128. The surgical tool 122 can be, for example, a pointer tool, a cutter tool, a drill guide, or any other tool that can be positioned within the surgical cavity 104. The surgical tool 122, or at least part thereof, can have been pre-inserted into the surgical cavity 104 prior to start of determining and/or tracking the one or more locations of interest. A user can position a distal end 138 of the surgical tool 122 at the location of interest 124. For example, the surgical tool 122 can be a pointer tool that includes a tip that can be positioned against location of interest 124. System 100 can determine a three-dimensional location of the distal end 138 of the surgical tool 122 based, at least in part, on a position and orientation of the fiducial marker 128 in the endoscopic images generated using the endoscopic imager 101, thereby determining the three-dimensional position of the location of interest 124 of the tissue 106.

The surgical tool 122 can include a button 132 or other user interface that a user can use to instruct the system 100 to determine the position of the location of interest 124. For example, the user can position the surgical tool 122 at or near the location of interest 124 and press the button 132 on the surgical tool 122 to indicate that the system 100 should determine the position of the location of interest 124. The surgical tool 122 can be connected to a tool controller 126 configured to control and/or operate the surgical tool 122. The tool controller 126 can receive a signal from the surgical tool 122 responsive to the button press. The tool controller 126 can send a notification to the image processing system 116 indicative of the user's instruction to determine the location of interest 124. The image processing system 116 can then analyze one or more endoscopic images to determine the three-dimensional position of the location of interest 124. The user can reposition the surgical tool 122 and provide another button press to control the system 100 to determine a new location of interest based on the repositioned position of the surgical tool 122. This can be repeated any number of times by the user.

As described further below, the image processing system 116 may track a position of the location of interest 124 relative to displayed endoscopic images over time and the tracked position of the location of interest 124 may be used to provide a visualization to a user associated with the location of interest 124. For example, a flag may be displayed, such as on display 118, at the location of interest 124 in endoscopic images displayed to the user to assist the user in keeping track of the location of interest 124. The image processing system 116 may track the position of the location of interest 124 based on estimates of motion of the endoscopic imager 101 relative to the determined position of the location of interest 124.

The image processing system 116 may use various methods for estimating motion of the endoscopic imager 101 relative to the determined position of the location of interest 124. In some examples, motion of the endoscopic imager 101 may be estimated based on motion data associated with motion of the medical imager. System 100 can include a motion sensor system, such as an inertial measurement unit (IMU) 144, for generating such motion data. The IMU 144 may be mounted to or incorporated in any suitable portion of the endoscopic imager 101, including, for example, included within the camera head 108, mounted to the camera head 108, or mounted to the endoscope 102 (e.g., mounted to a light post of the scope). The IMU 144 may include one or more accelerometers, gyroscopes, magnetic field sensors, and/or other sensors for sensing motion of the endoscopic imager 101. The IMU 144 may output motion data associated with motion of the endoscopic imager 101, such as translational accelerations in three dimensions, rotational position and/or acceleration in three dimensions, and/or magnetic field data. The motion data from the IMU 144 may be provided to the image processing system 116, such as via one or more wired or wireless links. For example, the motion data from the IMU 144 may be communicated via a wired link from the camera head 108 to the camera control unit 112 and from the camera control unit 112 to the image processing system 116 or may be transmitted wirelessly from the IMU 144 to the image processing system 116 or an intermediate communication system.

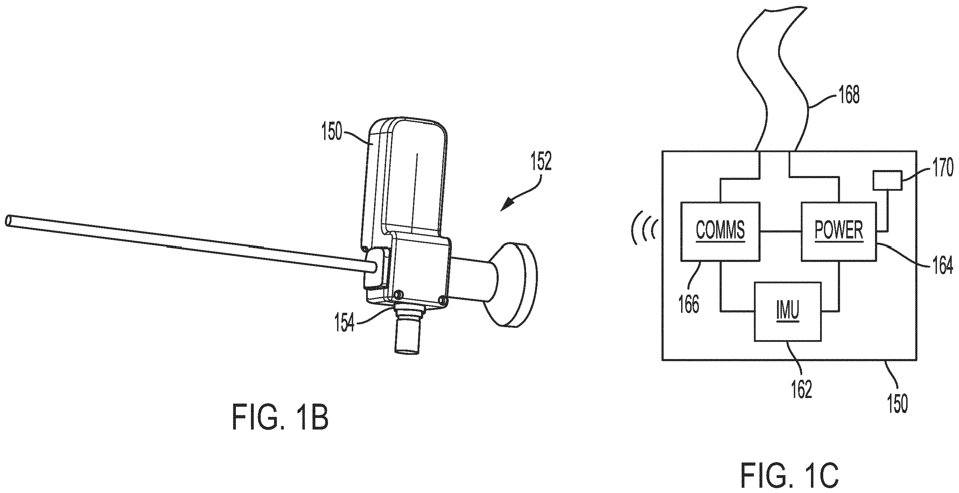

FIG. 1B illustrates an example of the mounting of an IMU package 150 to an exemplary endoscope 152. IMU package 150 can be used for IMU 144 of system 100. The IMU package 150 may be disposable or reusable. In the illustrated examples, the IMU package 150 is mounted to a light post 154 of the endoscope 152 such that the IMU package 150 is rigidly fixed to the endoscope 152. The IMU package 150 can be wired or wirelessly connected to an external system, such as image processing system 116.

FIG. 1C is an exemplary functional block diagram of the IMU package 150. The IMU package 150 can include an IMU 162, a power module 164, and a communication module 166. The power module 164 may receive power via cable 168 connected to an external power source. Alternatively, the power module 164 may be a wireless power module that includes a battery for storing power for powering the IMU package 150 between charges. The battery may be charged between uses, such as by connecting a power cable to the IMU package 150. Alternatively, a power harvesting module 170 may wirelessly harvest power. For example, power harvesting module 170 can include a photo diode that can generate electrical energy from light received from the light source 120, a portion of which may be directed via suitable optics within the light post 154 of the endoscope 152 to the power harvesting module 170. Alternatively, power harvesting module 170 can harvest power inductively when the IMU package 150 is placed on an inductive charger. The communication module 166 may transmit IMU data generated by the IMU 162 to an external computing system, such as image processing system 116. The IMU data may be transmitted via cable 168. Power and data may be transmitted over the same wires of cable 168 or over different wires or different cables. Alternatively, communication module 166 may transmit data wirelessly.

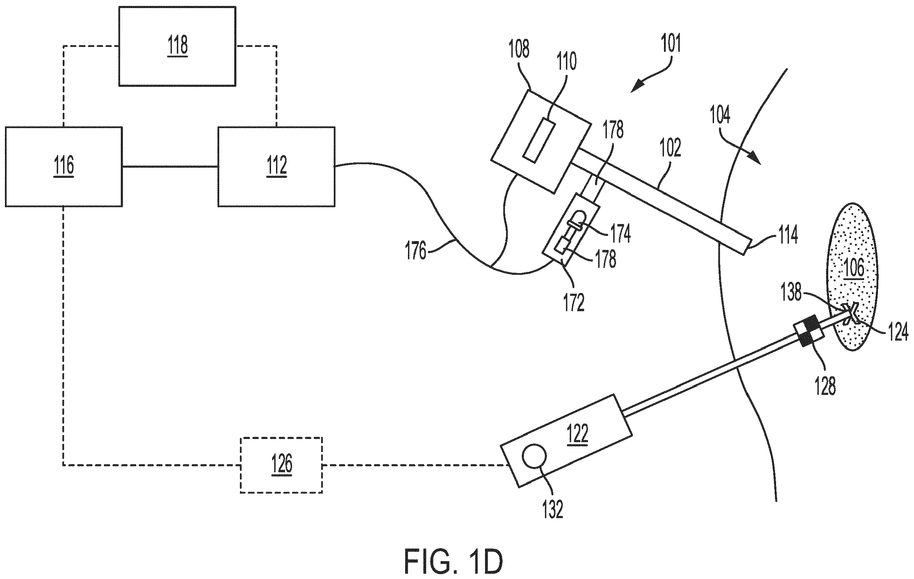

FIG. 1D illustrates a variation of system 100 of FIG. 1A in which an exemplary IMU package 172 includes one or more light sources 174 configured to generate the light for illuminating the surgical cavity 104 (e.g., instead of using light provided by light source 120 of FIG. 1A). The IMU package 172 is mounted to a light post 178 of the endoscope 102 and directs light generated by the one or more light sources 174 into the light post 178. IMU package 172 may be electrically connected to camera control unit 112 via a cable 176 (which may also connect to camera head 108). The cable 176 can provide power for an IMU 162 and the one or more light sources 174 and can provide for communication between the camera control unit 112 and the IMU 162 and light sources 174. For example, cable 176 may be or include a USB cable operable to provide power to IMU package 172 and enable communication between IMU package 172 and camera control unit 112. This can eliminate the need for the light cable, which is more cumbersome than a data/power cable.

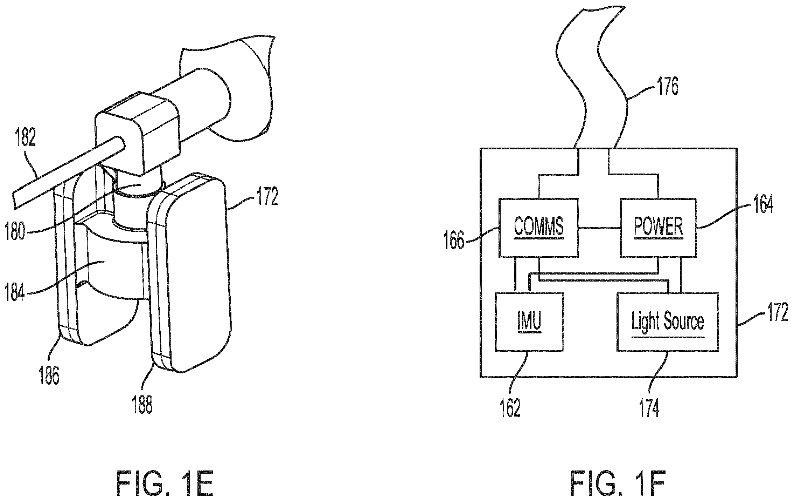

FIG. 1E illustrates an example of IMU package 172 connected to a light post 180 of an exemplary endoscope 182. One or more light sources (e.g., one or more LEDs, laser excited phosphor (LEP) lights, incandescent bulbs, and the like), may be positioned in a first portion 184 of the IMU package 172, an IMU may be positioned in a second portion 186 of the IMU package 172, and circuitry (e.g., control circuitry, communication circuitry, power circuitry, etc.) for the IMU and/or light sources may be positioned in a third portion 188 of the IMU package 172.

FIG. 1F is an exemplary functional block diagram of IMU package 172. Similar to IMU package 150 of FIG. 1C, IMU package 172 can include an IMU 162, a power module 164, and a communication module 166. IMU package 172 additionally includes the one or more light sources 174 for generating light provided via an endoscope to the surgical cavity 104. The one or more light sources 174 can include one or more LEDs for generating light suitable for illuminating a surgical cavity 104 and one or more optical components for combining and/or directing light from the one or more LEDs. The power module 164 may receive power via cable 176 connected to an external power source, such as camera control unit 112, and may provide power to the IMU 162 and the one or more light sources 174. The communication module 166 may transmit IMU data generated by the IMU 162 to an external computing system such as camera control unit 112 and/or image processing system 116 (e.g., via camera control unit 112). The communication module 166 may provide control signals to the one or more light sources 174 to control the light output by the light sources 174.

Returning to FIG. 1A, another method for generating motion data for estimating motion of the endoscopic imager 101 relative to the determined position of the location of interest 124 uses a camera-based tracking system 140, which may include at least one camera 142 that captures images of at least one tracker 146 of the endoscopic imager 101 to track a location of the endoscopic imager 101. For example, the tracker 146 could be or include one or more fiducial markers. The camera-based tracking system 140 may include a camera tracking processing unit 148 that may analyze images generated by the at least one camera 142 to determine the position and orientation (also commonly referred to as pose) of the endoscopic imager 101 based on the position and orientation of the at least one tracker 146. Motion data can be transmitted from the camera-based tracking system 140 to the image processing system 116. The motion data can be or include, for example, updated positions and/or orientations of the endoscopic imager 101 in any suitable reference frame, such as a global reference frame and/or a reference frame centered at camera 142. Estimates of motion of the endoscopic imager 101 can be generated based on motion data from multiple sources, such as from the IMU 144 (or multiple IMUs) and from the camera-based tracking system 140. Other tracking systems that have one or more sensors that are spaced apart from the endoscopic imager 101 may be used, including, for example, infrared tracking systems, electromagnetic field tracking systems, and/or any other tracking system suitable for tracking a location of the endoscopic imager 101.

In some examples, estimates of motion of the endoscopic imager 101 relative to the determined position of the location of interest 124 can be generated based on the endoscopic images generated by the endoscopic imager 101. For example, the image processing system 116 may analyze multiple endoscopic images captured at different points in time to determine motion of the imaged scene between the two points in time. The motion of the imaged scene can be used to estimate motion of the endoscopic imager 101 relative to the determined position of the location of interest 124. In some examples, the visual features in a single image are used to determine the pose of the endoscopic imager 101, and an estimate of motion of the endoscopic imager 101 is determined from differences in the determined pose over time (e.g., from one image to another). In some examples, structural light techniques are used to extract three-dimensional structure from an image for determining pose of the endoscopic imager 101. For example, the endoscopic imager 101 may include a lighting system that projects a pattern of light (e.g., a grid of pinpoint light) onto the scene and the warping of the pattern in an image can be used to determine three-dimensional information about the scene (e.g., depth information) from which the pose of the endoscopic imager 101 can be derived.

Such image-based endoscopic imager motion estimation may be combined with motion data-based endoscopic imager motion estimation to generate an estimate of the motion of the endoscopic imager 101. As described in more detail below, the estimate of the motion of the endoscopic imager 101 can be used to track the position of the location of interest 124 over time.

Although system 100 illustrates an exemplary endoscopic imaging system, it will be understood to a person of ordinary skill in the art that the principles described herein are not limited to endoscopic imaging but, rather, can be applied to any medical imaging system using any medical imager that may experience motion, including, for example, a hand-held open-field camera. As such, the IMU 144 or camera-based tracking system 140 can be used with any such medical imager. Further, while the methods described below often reference endoscopic imagers and endoscopic images, this is merely for illustration, and it should be understood that the same methods can be used for any medical images generated by any medical imagers.

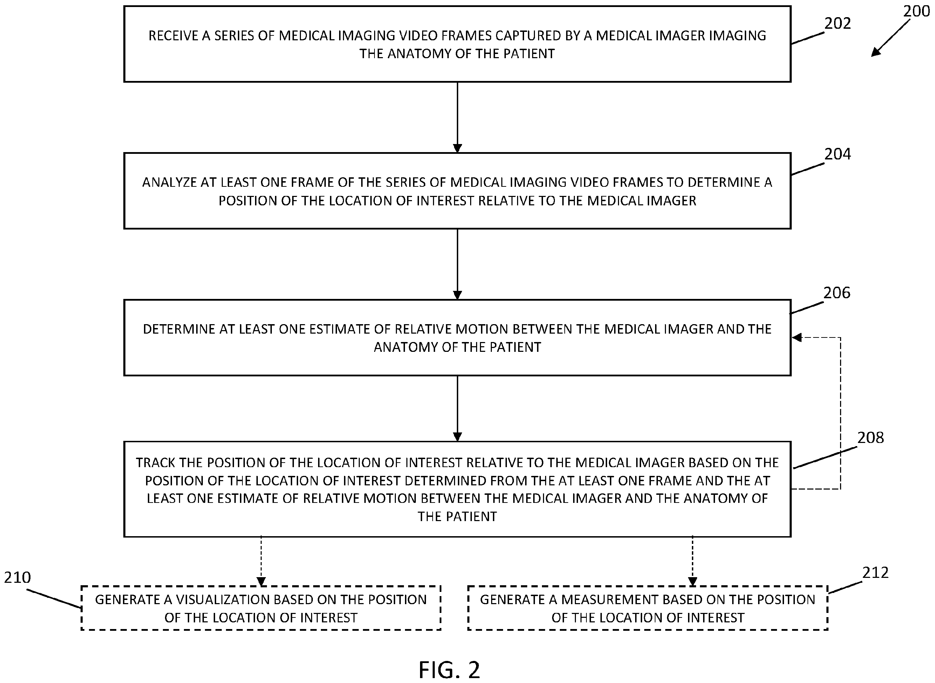

FIG. 2 is a flow diagram of an exemplary method 200 for tracking a location of interest of anatomy (anatomy can include bone, cartilage, ligaments, tendons, membranes, muscle, vessels, or any other anatomical tissue of the body) of a patient in endoscopic imaging. Method 200 can be performed by a suitable computing system configured thereto, such as, for example, image processing system 116 of system 100. Method 200 can be performed during a surgical procedure on a patient to provide guidance to one or more users (e.g., surgeons, medical staff, and the like) during the surgical procedure.

At step 202, a series of endoscopic images captured by an endoscopic imager imaging the anatomy of the patient may be received at the computing system. For example, image processing system 116 of system 100 may receive a series of endoscopic images from camera control unit 112 of system 100 based on endoscopic imaging data generated by endoscopic imager 101. The series of endoscopic images may be frames of endoscopic video and can include every frame generated by the camera control unit 112 or some subset of frames, such as every other frame, every third frame, etc. The endoscopic imager can be pre-inserted prior to start of method 200. The endoscopic image(s) capture anatomy of interest within a surgical cavity, such as tissue 106 within surgical cavity 104 of FIG. 1. The anatomy of interest may be, for example, bony anatomy.

At step 204, at least one image of the series of endoscopic images is analyzed by the computing system to determine a position of a location of interest of anatomy of a patient relative to an endoscopic imager. This analysis may be triggered, for example, by a user instructing the computing system to determine the position of a location of interest. For example, with reference to system 100 of FIG. 1, a user may have positioned the distal end 138 of surgical tool 122 at a location of interest 124 and press button 132 of surgical tool 122 to instruct the image processing system 116 to determine the position of the location of interest 124 relative to endoscopic imager 101. The computing system may analyze the endoscopic image that is being displayed at the time the user provided the input (or close in time to the input) instructing the computing system to determine the position of a location of interest. As noted above, the endoscopic image may capture anatomy of interest. The endoscopic image(s) may also capture a distal portion of one or more surgical tools located in the surgical cavity. For example, with respect to FIG. 1, a distal end 138 of surgical tool 122 may be located in the surgical cavity 104 within a field of view of the endoscopic imager 101. The distal end 138 of the surgical tool 122 may be positioned adjacent to (e.g., touching) the location of interest 124, such as for indicating a start point or end point for a measurement.

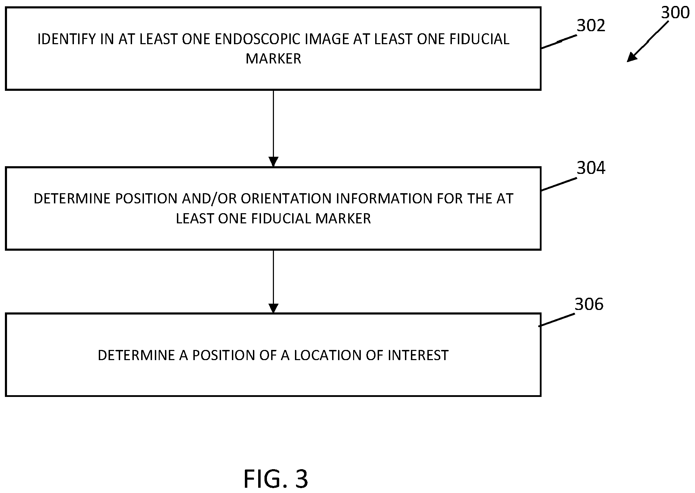

FIG. 3 is a flow diagram of an exemplary method 300 for analyzing at least one image of a series of endoscopic images to determine a position of a location of interest of anatomy of a patient relative to an endoscopic imager according to step 204 of method 200. Method 300 can be performed by a suitable computing system configured thereto, such as, for example, image processing system 116 of system 100. As noted above, the endoscopic image may capture a surgical tool. The surgical tool may include one or more fiducial markers that are captured in the endoscopic image. For example, with respect to FIG. 1, fiducial marker 128 may be within the field of view of endoscopic imager 101 such that the fiducial marker 128 is captured in endoscopic image(s) generated by the endoscopic imager 101. More than one fiducial marker may be captured in the endoscopic image(s). At step 302, at least one fiducial marker of a surgical tool is identified in the at least one endoscopic image. The fiducial marker can be identified by detecting one or more visual patterns of the fiducial marker and matching the one or more visual patterns to at least one fiducial marker pattern in a pre-defined list of fiducial marker patterns. The one or more visual patterns can be detected according to well-known image processing techniques, such as using a suitable edge detection algorithm and searching for edges that correspond to features of a pattern and/or using a machine learning model trained to identify one or more fiducial markers in endoscopic images.

Identification of the fiducial marker in step 302 may include extraction of information encoded by the fiducial marker. For example, the fiducial marker may be an ArUco marker that encodes an identity of the ArUco marker that may uniquely identify the ArUco marker relative to other ArUco markers such that the image processing system can access the predetermined spatial information associated with that ArUco marker (or the feature(s) of the surgical tool associated with that ArUco marker). The ArUco marker may additionally comprise encoded error detection and correction information such that any discrepancies and/or errors in detecting the bit pattern of the ArUco marker may be minimized to determine the correct information associated with a given ArUco marker. Other examples of fiducial markers that may be used are bar codes, Quick Response (QR) codes, glyphs, and AprilTags.

One or more machine learning models can be used to improve the accuracy of finding and identifying the fiducial markers with respect step 302. For example, a machine learning model can be used to segment the surgical tool in the endoscopic image. By segmenting the endoscopic image (i.e., determining the region of the endoscopic image that includes the surgical tool), an image processing algorithm can focus on the segmented region to search for the fiducial marker so that the fiducial marker can be more easily found than if the entire image were searched. In one or more examples, a sequence of endoscopic images (a sequence of endoscopic video frames) can be analyzed by one or more machine learning models so that, for instance, any temporal aspects of the video can be used by the one or more machine learning models to segment the surgical tool from an image and/or otherwise help to locate and identify the fiducial markers. In one or more examples, the one or more machine learning models can be an instance segmentation machine learning model configured to detect, segment, and classify individual objects in an image. An example of a suitable instance segmentation machine learning model that works on individual images is a Mask R-CNN. Examples of suitable instance segmentation machine learning models that work on video input and utilize the temporal component of the video are multi-scale spatio-temporal split (MS-STS) transformers and spatial-temporal graph neural networks.

At step 304, position and/or orientation information for at least a portion of the at least one fiducial marker in the at least one endoscopic image is determined. Predetermined information regarding the positions of features of a fiducial marker relative to one another can be used to calibrate the positions of the features of the fiducial marker in the endoscopic image (e.g., the pixel positions) such that the position and/or orientation of the fiducial marker or portions of the fiducial marker in a suitable reference frame can be determined. A suitable reference frame can be a camera-based reference frame defined as a coordinate system with a known origin located relative to the camera (e.g., camera head 108 of the endoscopic imager 101). Thus, the position and orientation of the fiducial marker or portion thereof may be determined relative to an origin located at the camera head. Examples of other reference frames that may be used include a tool-based reference frame and a world-based reference frame. A tool-based reference frame may have as its origin a particular location on, within, or otherwise relative to, for example, surgical tool 122, such as a center of a distal end 138 of the surgical tool 122. A world-based reference frame may have an arbitrarily defined origin that is fixed in world space.

Using a camera-based reference frame as an example, the positions of the corners of a fiducial marker in a camera-based reference frame can be determined based on the positions of the corners of the fiducial marker in the image and predetermined information regarding the positions of the corners of the fiducial marker relative to each other. With reference to system 100 of FIG. 1, the predetermined information regarding the fiducial marker may be stored in a memory of the image processing system 116 or may be stored in a memory of a computing device to which the image processing system 116 is communicatively connected, such as a memory of a hospital information network or a cloud-based storage. The predetermined information can be, for example, spacing between different features of the fiducial marker (e.g., spacing between corners of the fiducial marker) stored in a look-up table. The predetermined information regarding the fiducial marker may be retrieved from the memory by the image processing system based on information encoded in the fiducial marker. For example, a fiducial marker identification encoded in the fiducial marker may be cross referenced in a fiducial marker database to access the predetermined fiducial marker information associated with that particular fiducial marker. The predetermined information regarding the fiducial marker may then be used along with the position of the fiducial marker in the endoscopic image to determine the position and/or orientation of the fiducial marker or portions thereof in a camera-based reference frame (or other suitable reference frame). For example, the positions of the corners of a fiducial marker in a camera-based reference frame may be determined.

At step 306, a position of a location of interest in three-dimensional space is determined based on the position and/or orientation information for the at least one fiducial marker determined in step 304. The position of the location of interest can be determined, for example, by determining the position and/or orientation of a feature of the surgical tool in a suitable reference frame, such as a camera-based reference frame, and using the position and/or orientation of the feature of the surgical tool as the position of the location of interest. For example, with respect to FIG. 1, the position of the location of interest 124 in an endoscopic image can be determined by determining the position of the center of the distal end 138 of the surgical tool 122 in the endoscopic image based on the position and/or orientation information for the at least one fiducial marker, and the position of the center of the distal end 138 of the surgical tool 122 can be used as the position of the location of interest 124 in the endoscopic image.

The position of the feature of the surgical tool used to determine the position of the location of interest can be determined based on predetermined information associated with the location of the feature of the surgical tool relative to the one or more fiducial markers of the surgical tool. For example, the position of the center of the distal end 138 of the surgical tool 122 can be determined based on the position and orientation of fiducial marker 128 identified in the endoscopic image and the predetermined position of the center of the distal end 138 relative to the fiducial marker 128 or relative to one or more portions of the fiducial marker 128 that is stored in memory and accessed by the image processing system 116. The position of the center of the distal end 138 can be used as the position of the location of interest 124. With reference to system 100 of FIG. 1, the predetermined information associated with the location of the feature of the surgical tool relative to the one or more fiducial markers of the surgical tool may be stored in a memory of the image processing system 116 or may be stored in a memory of a computing device to which the image processing system 116 is communicatively connected, such as a memory of a hospital information network or a cloud-based storage.

Returning to FIG. 2, at step 206, at least one estimate of relative motion between the endoscopic imager and the anatomy of the patient is determined. The estimate of relative motion can be an estimate of the motion of the endoscopic imager relative to the anatomy of the patient from the point in time associated with the endoscopic image used to determine the position of the location of interest in step 204 to a later point in time, such as a point in time that a subsequent endoscopic image is received by the computing system (e.g., image processing system 116). The estimate of relative motion can include, for example, an amount of translation in each of three translational dimensions and an amount of rotation in each of three rotational dimensions. As described further below, the estimate of relative motion can be determined based on sensors that monitor motion of the endoscopic imager and/or based on image analysis.

The motion of the endoscopic imager can be monitored using one or more sensors configured to detect motion of the endoscopic imager. Exemplary sensors include accelerometer, gyroscopes, and magnetometers. In some examples, an IMU that includes one or more of these sensors is mounted to or incorporated into the endoscopic imager. For example, with reference to system 100 of FIG. 1, endoscopic imager 101 can include IMU 144, which can detect motion of the endoscopic imager and output motion data, such as linear accelerations in three dimensions, rotational velocities in three dimensions, and/or magnetic field measurements in three dimensions. The IMU 144 can include a processor running sensor fusion software to determine translations, rotations, and/or orientation from raw sensor data and can output this motion data to the image processing system 116. The IMU 144 can output the motion data on a periodic basis or in response to a query from the image processing system 116. Since IMU data is known to drift over time, the IMU may provide temperature readings and/or power consumption data to the image processing system 116 to compensate for drift. The image processing system 116 may use a suitable filter, such as a Kalman filter, a complimentary filter, a lowpass filter, and/or a bandpass filter, to programmatically remove drift.

Monitoring motion of the endoscopic imager using an IMU can include a calibration step. The calibration step can be performed, for example, at least once at the start of an imaging session. To calibrate the IMU, a user can place the endoscopic imager in a known orientation and provide an input indicating that the IMU can be calibrated. For example, the user can provide an input, such as a button press, to an IMU module mounted to the endoscopic imager, to the camera head 108, to the surgical tool 122, and/or to a user interface of the computing system. Upon receiving the user input, the readings from the IMU may be saved (e.g., by the image processing system 116) as a reference point.

The calibration step or a separate set-up step can include defining an offset associated with a difference in position between the IMU and a reference position on the endoscopic imager. For example, it may be desirable for IMU data to be associated with a tip of the endoscope 102 and an offset between the location of the IMU and the tip of the endoscope 102 may be used to compute a rigid transform between a reference frame centered at the IMU and a reference frame centered at the tip of the endoscope 102. Different endoscopic imager arrangements (e.g., different camera heads, different scopes, different camera couplers) may have different offsets. Thus, the calibration or set-up step can include a user providing an input (e.g., to the image processing system 116 or a user interface communicatively connected to the image processing system 116) associated with the endoscopic imager arrangement. The user input can be the offset itself or a selection of a preprogrammed option associated with the imager arrangement, wherein a predetermined offset for the imager arrangement has been stored. Alternatively, the offset could also be calculated during an optical calibration routine by generating and analyzing images of a calibration object of known size (e.g., a two-dimensional object displaying a pattern, or glyph, on one surface used for calibration).

In some examples, the one or more sensors used to detect motion of the endoscopic imager include one or more cameras of a camera-based tracking system that capture images of the endoscopic imager, specifically of a tracker located in fixed position relative to the endoscopic imager. For example, with reference to FIG. 1, camera-based tracking system 140 can include at least one camera 142 that captures images of at least one tracker 146 of the endoscopic imager 101 to track a location and orientation of the endoscopic imager 101. The camera-based tracking system 140 may include a camera tracking processing unit 148 that may analyze images generated by the at least one camera 142 to determine the position and orientation of the endoscopic imager 101 based on the position and orientation of the at least one tracker 146. Motion data can be transmitted from the camera-based tracking system 140 to the image processing system 116. The motion data can be, for example, a position and orientation of the endoscopic imager in a suitable reference frame, such as a world-based reference frame or a camera tracking system reference frame.

The image processing system 116 may receive the motion data from the IMU 144, the camera-based tracking system 140, and/or any other sensor-based motion monitoring system and can use the motion data to generate an estimate of relative motion between the endoscopic imager and the anatomy of the patient. The estimate of relative motion can be, for example, an amount of translation in the -x, -y, and/or -z direction and/or an amount of rotation about - x, -y, and/or -z axis. Optionally, the image processing system 116 may receive multiple motion data updates, accumulate the motion data updates in memory, and generate an estimate of relative motion between the endoscopic imager and the anatomy of the patient based on the accumulated motion data. For example, the image processing system 116 may generate an estimate of the relative motion for each frame of endoscopic video based on accumulated motion data that is received in the time period between frame captures. For example, multiple acceleration updates may be integrated to generate an estimate of linear translation. The image processing system 116 may generate an estimate of the relative motion based on a comparison between current motion data and motion data associated with the time at which the position of the location of interest was determined at step 304 shown in FIG. 3. For example, an angular orientation of the endoscopic imager 101 received from the IMU 144 may be compared to an angular orientation of the endoscopic imager 101 at the time that the location of interest 124 was determined in step 304 to determine an estimate of amount of change of the angular orientation of the endoscopic imager 101.

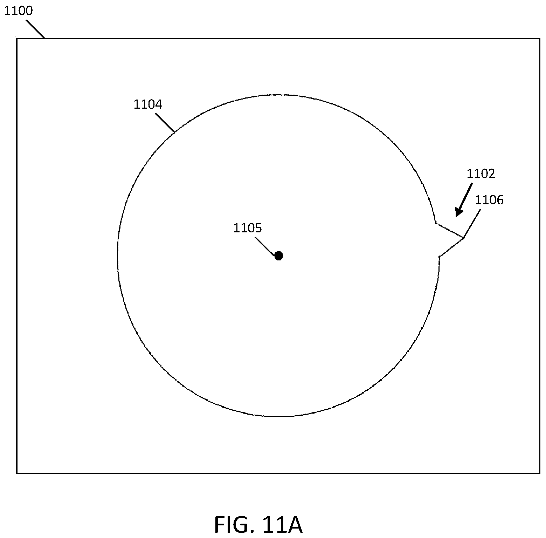

Determining an estimate of relative motion at step 206 can include determining a rotational orientation of endoscope 102 relative to the camera head 108 based on analysis of an endoscopic image. FIG. 11A illustrates an example of an endoscopic image 1100 having an endoscope rotational orientation indicator 1102 (also commonly referred to as a carrot or a notch). The position of the indicator 1102 about the circular perimeter of the field of view of the endoscopic image 1100 corresponds to a rotational orientation of the endoscope 102 relative to a camera head 108. The position of the indicator 1102 can change when the endoscope 102 is rotated about its longitudinal axis relative to the camera head 108, which a user may do to change to direction of view of the endoscopic imager 101 (for example, when the endoscope has an angled distal end). As such, the endoscopic image 1100 can be analyzed by the image processing system 116 to detect the indicator 1102 and determine its position about the circular perimeter 1104 of the field of view of the endoscopic image 1100, which provides the rotational orientation of the endoscope 102 relative to a camera head 108. Changes in the rotational orientation of the endoscope 102 relative to the camera head 108 can be included in the estimate of relative motion determined at step 206.

The image processing system 116 may determine the position of the indicator 1102 by processing the endoscopic image 1100 with one or more machine learning models trained to detect the circular perimeter 1104 of the field of view portion of the image 1100 and the indicator 1102. Once the circular perimeter 1104 is detected, the position of its center 1105 can be determined. The portion of the indicator 1102 that is furthest from the center 1105 (the tip 1106) can be located and its position is determined. In some variations, the indicator 1102 may project inwardly toward the center 1105, rather than outwardly away from the center 1105, and the portion of the indicator 1102 that is closest to the center 1105 may be located and its position determined. The field of view portion of the image may be divided into angular regions (e.g., from 0 to 360 degrees) centered at the center 1105 of the circular perimeter 1104. The angular region that includes the location of the indicator 1102 may provide the rotational position of the indicator 1102 about the circular perimeter 1104. The rotational position of the indicator 1102 about the circular perimeter 1104 may be used as the rotational orientation of the endoscope 102 relative to the camera head 108.

Determining an estimate of relative motion at step 206 can include comparing the rotational orientation of the endoscope 102 relative to the camera head 108 in one endoscopic image to a rotational orientation of the endoscope 102 relative to the camera head 108 in a previous endoscopic image. This can provide an estimate of the relative rotational motion of the endoscope 102 relative to the camera head 108 between the two endoscopic images. The estimate of the relative rotational motion of the endoscope 102 between the two endoscopic images can be used in combination with motion data generated by an IMU (or motion data from any other motion estimation technique described herein). For example, motion of the camera head 108 can be determined based on an IMU mounted to the camera head 108 (which may not detect motion of the endoscope 102 relative to the camera head 108) and this motion can be combined with the estimate of the rotational motion of the endoscope 102 relative to the camera head 108 determined from the endoscopic image to determine an estimate of a total motion of the endoscope 102 (and, thereby, the field of view). Alternatively, the IMU may be configured to detect rotation of the endoscope 102 relative to the camera head 108 (for example, an IMU may be mounted to the endoscope 102, such as IMU package 150 of FIG. 1B), and the estimate of the rotational motion of the endoscope 102 relative to the camera head 108 determined from the endoscopic image can be combined with a motion estimate from the IMU data (for example, using a suitable sensor fusion algorithm) or can be used as a check on the motion estimate from the IMU data (including, being used instead of the motion estimate from the IMU data).

The rotational angle of the endoscope 102 relative to the camera head 108 can additionally, or alternatively, be used in calibrating the endoscopic image 1100. For example, the rotational angle of the endoscope 102 can be used in applying a calibration matrix to the endoscopic image 1100.

Analysis of the endoscopic image can be used, additionally or alternatively, to determine deflection of the endoscope 102, which may occur when a user applies a force of sufficient magnitude to the endoscope 102 in a direction transverse to a longitudinal axis of the endoscope 102. An estimate of relative motion of the endoscopic imager can account for a determined amount of deflection of the endoscope 102, such as by combining a motion of the camera head 108 with a deflection of the endoscope 102 to determine a motion of the distal end of the endoscope 102. Additionally, or alternatively, a determined amount of deflection of the endoscope 102 can be compared to a predetermined threshold and a warning may be provided to the user that deflection of the endoscope 102 is excessive and accuracy of the tracking the location of interest of anatomy may be low. Determining the amount of deflection can be performed by a suitable computing system configured thereto, such as, for example, image processing system 116 of system 100.

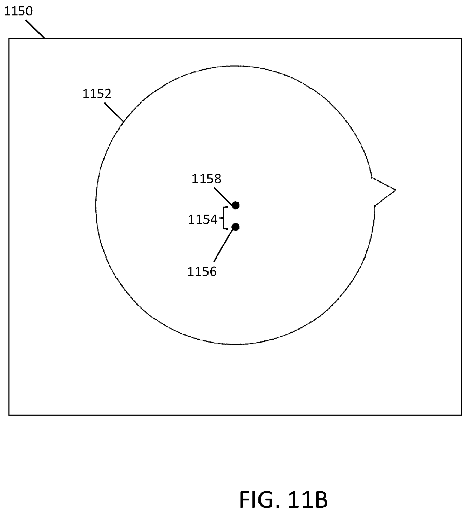

Deflection of the endoscope 102 can be determined by determining that the field of view portion of the endoscopic image is shifted off-center in the endoscopic image. FIG. 11B illustrates an example of an endoscopic image 1150 in which the field of view portion of the endoscopic image 1100 (defined by circular perimeter 1152) is shifted upward, as indicated by the distance 1154 between a center 1156 of the endoscopic image 1150 and a center 1158 of field of view portion of the endoscopic image 1100. The center 1158 of field of view portion of the endoscopic image 1100 can be determined by detecting the circular perimeter 1152 (such as using a suitable machine learning algorithm as discussed above) and determining its center. The location of the center 1158 of field of view portion of the endoscopic image 1100 can be compared to the location of the center 1156 of the endoscopic image 1150 to determine whether the field of view portion is shifted. Optionally, an amount of shift (i.e., distance 1154) may be compared to a predetermined threshold and a warning may be provided to the user of low accuracy of the tracking the location of interest of anatomy if the amount of shift is too great. Additionally, or alternatively, the amount of shift can be used in the estimate of relative motion of the endoscopic imager. For example, empirical testing can be used to generate a function (or lookup table) that relates an amount and direction of shift of the field of view portion in the endoscopic image to an amount and direction of deflection of a given endoscope (or given type of endoscope). The function or lookup table can then be used to determine the deflection of the endoscope 102 for a given amount of shift of the field of view portion of the endoscopic image. In some variations, deflection of the endoscope 102 is sensed by one or more sensors (e.g., strain gauges) of the endoscope 102. The determined deflection can then be combined with motion of the camera head 108 to determine a motion of the distal end of the endoscope 102.

The estimate of relative motion of the endoscopic imager relative to the anatomy of interest can be determined, additionally or alternatively, based on monitoring motion of the field of view of the camera. Pairs of endoscopic images can be used to determine how the field of view shifted between the endoscopic images and this shift can be used to estimate the motion of the endoscopic imager relative to the anatomy of the patient.

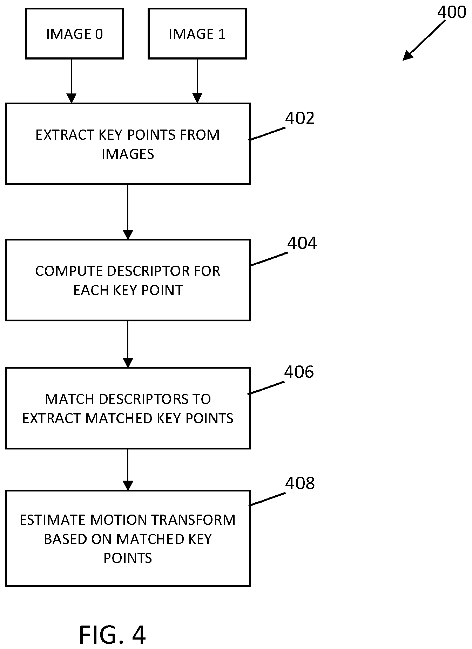

FIG. 4 is a flow diagram of an exemplary method 400 for estimating relative motion of the endoscopic imager relative to the anatomy of the patient, according to step 206 of method 200, based on processing pairs of images using visual odometry. Method 400 can be performed on any pair of endoscopic images (labeled image0 and image1 in FIG. 4). Method 400 can be performed by a suitable computing system configured thereto, such as, for example, image processing system 116 of system 100. At step 402, each of the images is analyzed to extract key points, or the locations of the most distinctive features (e.g., edges) in the images, using any suitable key point detector, such as a Harris detector or a Scale-Invariant Feature Transform (SIFT). At step 404, descriptors are generated for each key point extracted from the images. The descriptors are representations of visual characteristics of the key point or region of the key point that may be insensitive or less sensitive to image capture-based variability, such as rotations and illumination changes. The same algorithm, such as the SIFT algorithm, may provide both key point extraction and descriptor generation. At step 406, the descriptors for the two images are compared to identify matched key points, such as using a nearest neighbor search or any other suitable matching algorithm. The matched key points are the pixel locations in each image that correspond to the same feature captured in the pair of images. At step 408, the matched key points are used to generate an estimate of motion of the endoscopic imager relative to the anatomy of the patient. The estimate of motion can include, for example, translation in one or more dimensions and rotation in one or more dimensions.

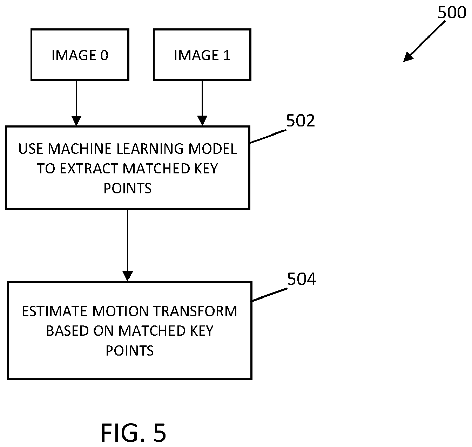

Another approach to estimating motion of the endoscopic imager relative to the anatomy of the patient from pairs of images that can be used for step 206 of method 200 includes using a machine learning model that takes as input a pair of images and generates as output a set of matched key points for the images. This is illustrated in FIG. 5, which is a flow diagram of a method 500 that can be used for step 206 of method 200. Method 500 can be performed by a suitable computing system configured thereto, such as, for example, image processing system 116 of system 100. At step 502, a machine learning model processes a pair of images-image0 and image 1-and outputs a set of matched pairs of key points. At step 504, the matched key points are used to generate an estimate of motion of the endoscopic imager relative to the anatomy of the patient in similar fashion to step 408 of method 400. This can be used as the estimate of relative motion according to step 206 of method 200.

The machine learning model used in step 502 can be a transformer-based matching model that is trained using images captured by a camera with viewpoint changes and/or illumination changes and camera positional information corresponding to the position of the camera for each image. Examples of suitable machine learning models include Local Feature TRansformer ("LoFTR"), MatchFormer, QuadTreeAttention, and TransMatcher.

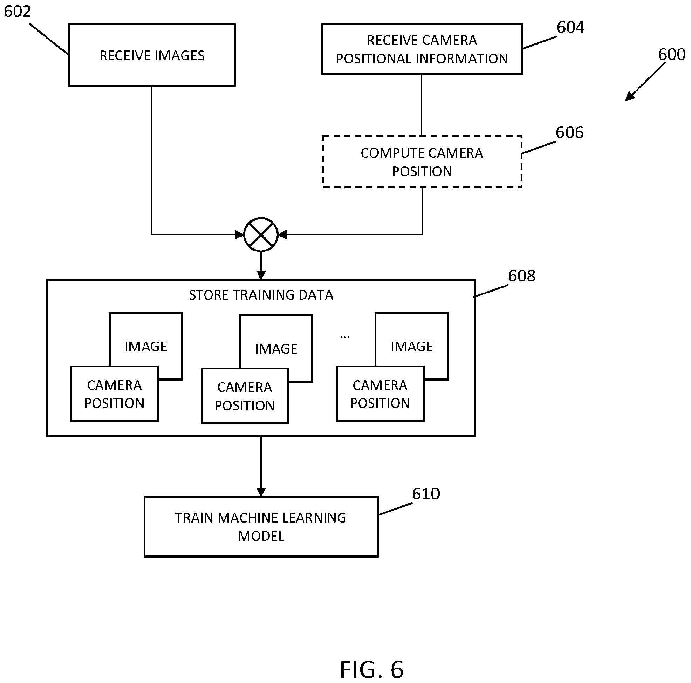

FIG. 6 illustrates a method 600 for training a machine learning model usable for step 502 of method 500. Method 600 can be performed by a computing system, such as image processing system 116 of system 100 of FIG. 1A or any other suitable computing system. At step 602, images captured using a suitable camera, such as endoscopic imager 101 of system 100, are received by the computing system. The images can include, for example, endoscopic images, which may include endoscopic images of anatomy corresponding to the anatomy of the patient for which method 200 is performed. The images can include images generated during medical procedures on patients, images generated using cadavers and/or animal models, non-medical images, and any combination of these types of images.

At step 604, camera positional information for the camera used in step 602 is received by the computing system. Techniques similar to those used for step 204 of method 200 can be used to generate this positional information. For example, camera positional information can be generated using an IMU mounted to the camera, in similar fashion to IMU 144 mounted to endoscopic imager 101 of system 100 of FIG. 1, and/or using a camera-based tracking system, such as camera-based tracking system 140 of system 100 of FIG. 1. At step 606, camera position and orientation may be computed from the camera positional information. For example, the computing system may derive camera position and orientation from camera motion data received from an IMU fixed to the camera. In some examples, the camera positional information received from, for example, the camera-based tracking system, is camera position and orientation (for example, the camera-based tracking system may provide absolute position and orientation of the camera) such that the computing system need not derive camera position and orientation, and step 606 may include transforming the camera position and orientation received from the camera-based tracking system from a reference frame used by the camera-based tracking system to a desired reference frame. In some examples, the position and orientation received from the camera-based tracking system is usable directly and step 606 is not performed.

In some examples, the training images are synthetic images (e.g., single synthetic snapshot image or synthetic video frames) generated using a synthetic image generation program that can simulate a scene from different simulated camera viewpoints. In these examples, the simulated camera position and orientation can be queried from the program and used as synthetic positional data for the simulated camera for training. A process for generating synthetic images can begin by retrieving one or more models of bones of a j oint from a database of bones of a joint segmented from CT scans and generating one or more three-dimensional models of the joint in different states (e.g., healthy, injured, repaired). The one or more three-dimensional models may include representations of the bones and representations of soft tissue around the bones. The three-dimensional models of the joint are then used to generate simulated endoscopic scenes of the joint, which may include representations of surgical tools generated from three-dimensional models of the tools. One or more synthetic images are then created by generating a two-dimensional rendering of the scene from different simulated camera perspectives, and synthetic positional data is extracted. This synthetic positional data can include camera pose, depth maps, and optical flow.

At step 608, the images and corresponding camera position and orientation are associated and stored as a training data set. At step 610, the training data set is used to train the machine learning model. The camera position and orientation can be used to generate ground truth matches used for supervision of the machine learning model during training.

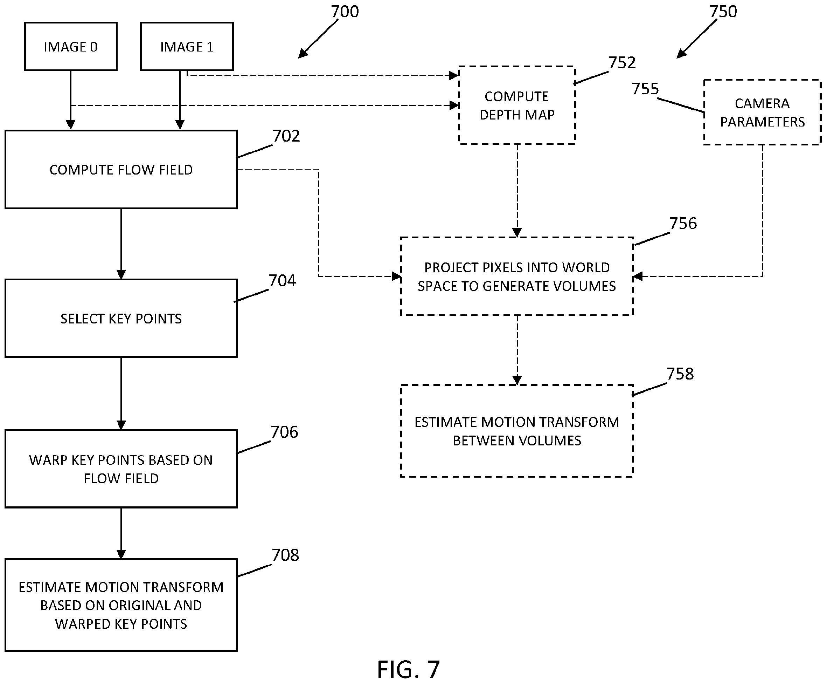

Yet another approach to estimating motion of the endoscopic imager relative to the anatomy of the patient from pairs of images that can be used for step 206 of method 200 uses optical flow, which produces a flow field (pixel motion vectors) from a pair of input images that estimates the apparent motion of pixels in the image. An exemplary method 700 of using optical flow to estimate motion of the endoscopic imager relative to the anatomy of the patient from pairs of images is shown in FIG. 7. Method 700 can be performed by a suitable computing system configured thereto, such as, for example, image processing system 116 of system 100.

At step 702, an optical flow model computes a flow field for a pair of images-image0 and image1. Any suitable optical flow model may be used. Optical flow can be done sparsely, where flow information is computed only for points of interest, or densely, where flow information is computed for every pixel in the input image pair. Examples of suitable algorithms for dense flow calculation include Farneback and TV-L1, and examples of suitable algorithms for sparse flow calculation include Lucas-Kanade and Sparse RLOF. In the sparse case, the points of interest can be or include points that a user has chosen to interact with previously, such as points determined in step 204 of method 200. In some examples, the optical flow model is a machine learning model, such as Recurrent All-pairs Field Transformations ("RAFT"), which generates a dense flow field. The machine learning model may be trained using method 600 of FIG. 6. In some examples, a RAFT model can be first trained in a supervised training step on a synthetic training dataset (e.g., synthetic images and synthetic positional data) or labeled training images (e.g., from a different domain, including publicly available labeled training images) and then adapted to processing real surgical images of a target domain without requiring training on labeled images for the target domain. An example of such a framework is UnDAF (A General Unsupervised Domain Adaptation Framework for Disparity or Optical Flow Estimation).

At step 704, several key points are selected. The key points can be selected from the images at random (e.g., in examples in which a dense flow field has been generated) or can be selected using a key point detector, such as described above with respect to step 402 of method 400. Optionally, a key point detector can be restricted to detecting key points only in areas corresponding to portions of the flow field with the highest confidence levels (as determined by the optical flow model). At step 706, the flow field is indexed at the selected key points and used to warp the sampled key points. At step 708, the original key points and the warped key points are processed by a motion estimation algorithm to estimate camera motion in similar fashion to step 408 of method 400. This can be used as the estimate of relative motion according to step 206 of method 200. Optionally, steps 706 and 708 are performed to estimate two-dimensional motion, which may be suitable when motion of the medical imager and/or the anatomy of the patient is relatively small. As such, the estimate of relative motion according to step 206 of method 200 can be an estimate of two-dimensional relative motion.

FIG. 7 also illustrates an alternative method 750 for estimating camera motion using the flow field. At step 752, depth maps are computed for each of the images. The depth map for each image may be generated by a machine learning model trained to determine relative depths in images. Alternatively, depth maps and camera movement trajectories can be estimated simultaneously by using a multi-information joint learning machine learning model that is trained in a self-supervised regime on monocular arthroscopy frames from a video sequence. Such a machine learning model may provide high accuracy and high training efficiency (since it does not require human provided labels due to self-supervision). Such a multi-information joint learning machine learning model may allow for accurate motion estimation for a large range of camera movements. The depth map may include an array of relative depth values that may include a relative depth value corresponding to each pixel of the image. The depth map may include, for example, depth values ranging from 0 to 1 where values closer to 1 are associated with shallower locations (relative to the camera) and values closer to 0 are associated with deeper locations (or vice versa).

At step 756, each pixel for each of the images is projected into world space (i.e., three-dimensional positions are determined for each pixel) to generate a three-dimensional volume for each image. The pixels are projected using the flow field computed in step 702, the depth maps computed in step 752, and one or more parameters 755 associated with the camera, such as focal length. For example, a plurality of pixels of a first image can be selected, the flow field for those pixels can be used to identify the corresponding pixels in the second image, and the relative positions of the pixels in the two images can be used along with the depth maps computed in step 752 and the intrinsic matrix of the camera as an input to a three-dimensional space transformation algorithm. At step 756, a matching algorithm, such as a cost function, is used to estimate a motion transformation between the three-dimensional volumes associated with the images. This can be used as the estimate of relative motion according to step 206 of method 200.

Returning to method 200 of FIG. 2, at step 208, the position of the location of interest is tracked based on the position of the location of interest determined from the at least one frame in step 204 and at least one estimate of relative motion between the medical imager and the anatomy of the patient determined in step 206. Tracking the position of the location of interest can include updating a stored position of the location of interest determined in step 204 based on motion of the camera determined in step 206. For example, if the camera moved in an x- direction by an amount d, the x- position of the location of interest can be updated by decrementing its x- positional value by the amount d.

Steps 206 and 208 can be repeated, such as to update the position of the location of interest for a more recently captured video frame. For example, steps 206 and 208 can be repeated for each new image received in step 202. As described above, a plurality of different methods may be used in step 206 to generate the estimate of the relative motion of the endoscopic imager. Optionally, different methods can be used at different times. For example, an image analysis-based motion estimate method, such as any of methods 400, 500, and/or 700, which may be computationally intensive, may be used less frequently than a frame rate (every Mth frame, where M is greater than one) and a sensor-based (e.g., IMU or camera-based tracking) motion estimate method may be used more frequently than the image analysis-based motion estimate method (e.g., every Nth frame, where N is less than M). Using multiple methods in this way may be useful to leverage the different strengths of different methods. For example, since IMU sampling rates are generally relatively high and the computing resources required to compute camera motion estimates from the IMU data is typically quite low, IMU-based camera motion estimation may be performed at high rates and on demand. However, as noted above, IMU data is known to suffer from drift and, therefore, may be less reliable when used to track motion over long periods. Image analysis-based methods, such as method 400, do not suffer from drift but may be so computationally intensive that there may be a multiframe lag between when an image is received and when the camera motion estimate for that image is determined. To leverage the different strengths of these two methods, an IMU-based camera motion estimate may be used, for example, on every frame, and method 400 may be used every several frames to correct for the drift associated by the IMU-based camera motion estimates. Different image-based methods may be used in similar fashion. For example, an optical flow-based method, such as method 700 may be less computationally intensive than the machine learning model-based method 500 and, as such, method 700 may be used in combination with method 500, with method 700 being used at a higher rate than method 500.

More than one method for estimating the relative motion of the endoscopic imager can be used at the same time and the different estimates (or different data used to generate the estimates) can be combined in any suitable fashion to generate a higher confidence estimate. The different estimates or different data used to generate the estimates can be combined using, for example, a Kalman filter, which can be programmed with transfer functions, expected variance, and frequency for each data source and is capable of producing a maximum likelihood estimate at each time step. For example, a weighting function for combining motion estimates can include weights that correspond to the expected variance of a given data source, such as weights that are based on the standard deviation of the data source. For example, one or more accelerometers, gyroscopes, magnetic field sensors, and/or other sensors of IMU 144 may each be associated with a respective standard deviation. In this example, the respective standard deviation associated with each component of IMU 144 may be aggregated, or averaged, to determine a weight indicating the expected variance of the IMU 144 data source. Any combination of the methods for estimation camera motion can be combined in this way. Using multiple different camera motion estimation methods can also be useful for detecting out of bounds conditions for which location of interest positional tracking may not be useable or reliable. For example, gross motion of the patient may result in unreliable motion estimates from an image-based camera motion tracking (because of the relatively large movement of the field of view likely associated with gross patient motion), and in such an instance, it may be desirable to stop tracking the location of interest (and clear the location of interest altogether) because the patient may not be returned to the same position. Since the motion estimate from sensor-based camera motion tracking will not show a corresponding movement (because the patient moved, not the camera), a delta between the image-based camera motion tracking and sensor-based camera motion tracking that is above a threshold can be used to stop the tracking process. Optionally, an alert may be provided to the user indicating that tracking has stopped, and that the user should reselect a location of interest for tracking.

Whether and/or to what extent image-based motion estimates are used in combination with other motion estimates may be based on one or more metrics associated with the quality of the image(s). For example, a blur metric (and/or any other image quality metric) may be generated for images, and the value of the blur metric may be factored into weights assigned to motion estimates derived from the images. Additionally, or alternatively, an image quality metric, such as a blur metric, can be compared to an image quality threshold and, if the threshold is not met, motion estimates derived from the low-quality images can be discarded.

Returning to FIG. 2, method 200 may include optional step 210 of generating a visualization (e.g., by image processing system 116) that includes a graphical indication of the location of interest positioned in the visualization relative to the images received in step 202 according to its tracked position. The visualization may be displayed on any suitable display, such as display 118 of system 100. The visualization may be updated over time to reposition the graphical indication of the location of interest according to the tracking of the position of the location of interest.

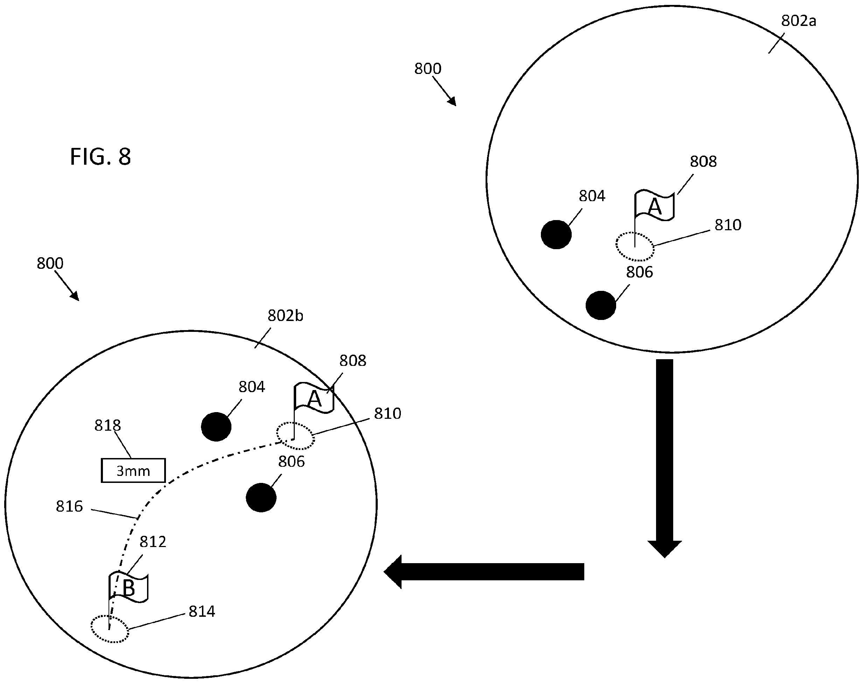

FIG. 8 illustrates an exemplary visualization of anatomy of interest in which one or more graphical indications of one or more locations of interest are relocated in the visualization based on the movement of the endoscopic imager such that the graphical indication(s) continues to accurately indicate the position of the location(s) of interest relative to the displayed scene. FIG. 8 illustrates two separate instantiations of a visualization 800, each including a different frame 802a and 802b of an endoscopic video. Frame 802a was taken earlier in time than frame 802b. Frames 802a and 802b can capture features 804 and 806 within the field of view. These features 804 and 806 may be identified as key points in step 402 of method 400, step 502 of method 500, or step 704 of method 700. Visualization 800 can include a flag 808 that indicates a location of interest 810. The position of the flag 808 in the visualization 800 for frame 802a may be determined according to method 300 of FIG. 3. Thus, while features 804 and 806 represent parts of the scene that are visible within the frame 802a, flag 808 is a graphical feature overlayed on the frame 802a.

If the endoscopic imager moves, then the position of features 804 and 806 will move within the visualization, based on the direction and distance that the endoscopic imager moves. For instance, as shown in FIG. 8, frame 802b can represent a frame that would appear if the endoscopic imager was moved such that the field of view is further leftward and downward than the field of view of frame 802a. In such an example, the position of features 804 and 806 will be further upward and rightward in frame 802b than in frame 802a. Such motion of the endoscopic imager relative to the imaged tissue is estimated according to step 206 of method 200, such as based on the differences in position of features 804 and 806 in 802a and 802b or based on camera motion sensor data, and used to update the position of the location of interest in step 208 of method 200. The position of the location of interest updated according to step 208 may be converted from the reference frame in which it is tracked (e.g., a camera-based reference frame) and to an image-based reference frame using a suitable transform and the position of the flag 808 in the visualization 800 of frame 802b is be moved further upward and leftward accordingly.

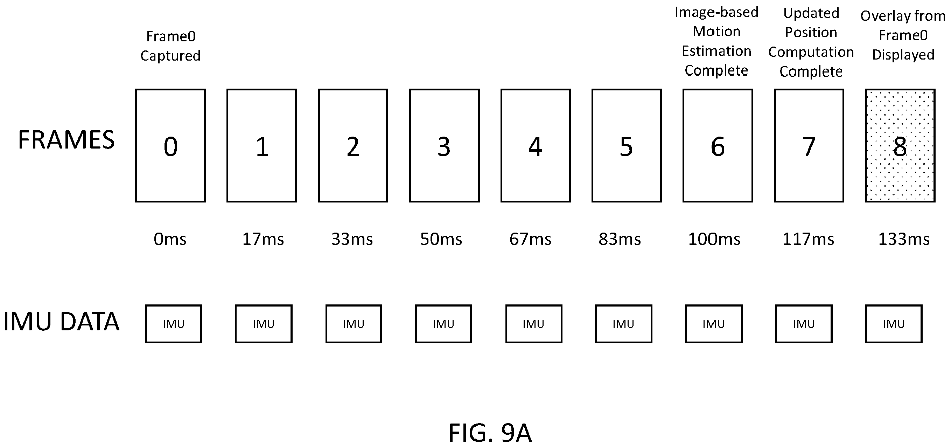

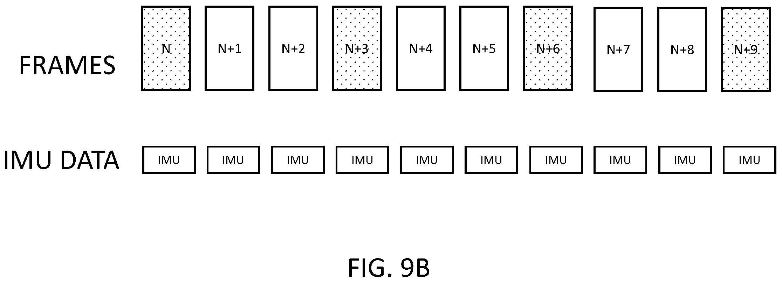

FIGS. 9A and 9B illustrate exemplary schemes for tracking the position of a location of interest and updating a visualization according to the tracked position of the location of interest according to various examples of method 200 of FIG. 2. FIG. 9A illustrates exemplary timing for generating an estimate of the relative motion between the endoscopic imager and the anatomy of the patient using an image-based camera motion estimation method, such as method 400, and using that estimate to update a position of a location of interest in a displayed visualization. In the example of FIG. 9A, video is captured at a frame rate of about 60 frames per second. A new frame (frame0) is captured at time 0. Frame0 and a previously captured frame are processed using method 400 to generate an estimate of the relative motion of the endoscopic imager, which takes about 100ms. The motion estimate is used to generate an updated position of a location of interest, which in this example takes another 17ms. The updated position is then available for use to generate a visualization in which a position of the graphical indication of the location of interest is updated and overlayed on frame8. Thus, in this example, the update for the position of the location of interest generated solely based on the image-based analysis of method 400 may be delayed by the equivalent of 8 frames. To account for this delay, IMU data may be collected in the time that it takes to generate the image-based motion estimate and a motion estimate from the IMU data may be added to the image-based motion estimate to generate a motion estimate that is contemporaneous with frame8, resulting in a more accurate placement of the graphical indication of the location of interest.

FIG. 9B illustrates an example in which an image-based camera motion estimation method, such as method 400, is used less often than every frame, and an IMU-based camera motion estimation method is used every frame. The image-based camera motion estimation method may be used on every third frame such that the visualization for every third frame (*N*, *N+3, N+6, N*+9) is based on an image-based camera motion estimate (for example, using the timing of the example of FIG. 9A, the visualization using frame *N* is based on an image-based camera motion estimate from frame *N-*8) with or without IMU-based adjustment. The visualization is updated for the interim frames (e.g., *N*+1*, N*+2*, N*+4*,* etc.) using only the IMU data. So, the visualization for frame *N*+1 may be based on the position of the location of interest determined for the visualization of frame *N* updated based on the camera motion estimate from the IMU data received from the time of frame *N* to the time of frame *N*+1. Thus, the computationally intensive image-based camera motion estimate may be used less frequently while maintaining accurate tracking and graphical representation of the location of interest.

Returning to FIG. 2, method 200 may include an optional step 212 of generating one or more measurements based on the position of the location of interest determined and tracked according to steps 202-208. Subsequent to the user specifying the first location of interest, the user may provide an input instructing the computing system to determine a position of a second location of interest at a later point in time, such as by moving the surgical tool 122 to a different location of the tissue 106 and providing an input (e.g., a button press) indicating that the user would like a measurement from the first location to the second location. In response, the computing system (e.g., image processing system 116) may determine the position of the second location of interest according to step 204. The computing system may then generate a measurement of the distance from the first location of interest (as tracked according to motion of the endoscopic imager between the time the first location of interest was first identified to the time the second location of interest is identified) to the second location of interest. The measurement may be included in a visualization, such as visualization 800. An example of this measurement is illustrated in FIG. 8 in which a second location of interest 814 has been specified, as indicated by flag 812. A measurement 818 of the distance 816 from the second location of interest 814 to the first location of interest 810 may be displayed in the visualization 800. Additionally, or alternatively, one or more screenshots of the locations of interest 810, 814 and the distance 816 between them can be saved for later use. For example, the one or more screenshots can be viewed by a user (e.g., a surgeon) at a conclusion of a procedure or at a later time and/or one or more screenshots (e.g., a selected number of screenshots) can be included in a post-operative report.

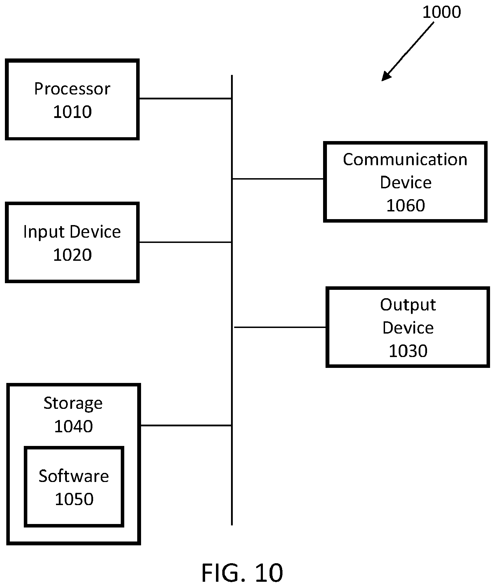

FIG. 10 illustrates an example of a computing system 1000 that can be used for one or more components of system 100 of FIG. 1, such as one or more of camera head 108, camera control unit 112, camera tracking processing unit 148, light source 120, tool controller 126, and image processing system 116. System 1000 can be a computer connected to a network, such as one or more networks of hospital, including a local area network within a room of a medical facility and a network linking different portions of the medical facility. System 1000 can be a client or a server. System 1000 can be any suitable type of processor-based system, such as a personal computer, workstation, server, handheld computing device (portable electronic device) such as a phone or tablet, or dedicated device. System 1000 can include, for example, one or more of input device 1020, output device 1030, one or more processors 1010, storage 1040, and communication device 1060. Input device 1020 and output device 1030 can generally correspond to those described above and can either be connectable or integrated with the computer.

Input device 1020 can be any suitable device that provides input, such as a touch screen, keyboard or keypad, mouse, gesture recognition component of a virtual/augmented reality system, or voice-recognition device. Output device 1030 can be or include any suitable device that provides output, such as a display, touch screen, haptics device, virtual/augmented reality display, or speaker.

Storage 1040 can be any suitable device that provides storage, such as an electrical, magnetic, or optical memory including a RAM, cache, hard drive, removable storage disk, or other non-transitory computer readable medium. Communication device 1060 can include any suitable device capable of transmitting and receiving signals over a network, such as a network interface chip or device. The components of the computing system 1000 can be connected in any suitable manner, such as via a physical bus or wirelessly.

Processor(s) 1010 can be any suitable processor or combination of processors, including any of, or any combination of, a central processing unit (CPU), field programmable gate array (FPGA), and application-specific integrated circuit (ASIC). Software 1050, which can be stored in storage 1040 and executed by one or more processors 1010, can include, for example, the programming that embodies the functionality or portions of the functionality of the present disclosure (e.g., as embodied in the devices as described above), such as programming for performing one or more steps of method 200 of FIG. 2, method 300 of FIG. 3, method 400 of FIG. 4, method 500 of FIG. 5, method 600 of FIG. 6, and/or method 700 and 750 of FIG. 7.

Software 1050 can also be stored and/or transported within any non-transitory computer-readable storage medium for use by or in connection with an instruction execution system, apparatus, or device, such as those described above, that can fetch instructions associated with the software from the instruction execution system, apparatus, or device and execute the instructions. In the context of this disclosure, a computer-readable storage medium can be any medium, such as storage 1040, that can contain or store programming for use by or in connection with an instruction execution system, apparatus, or device.

Software 1050 can also be propagated within any transport medium for use by or in connection with an instruction execution system, apparatus, or device, such as those described above, that can fetch instructions associated with the software from the instruction execution system, apparatus, or device and execute the instructions. In the context of this disclosure, a transport medium can be any medium that can communicate, propagate, or transport programming for use by or in connection with an instruction execution system, apparatus, or device. The transport computer readable medium can include, but is not limited to, an electronic, magnetic, optical, electromagnetic, or infrared wired or wireless propagation medium.

System 1000 may be connected to a network, which can be any suitable type of interconnected communication system. The network can implement any suitable communications protocol and can be secured by any suitable security protocol. The network can comprise network links of any suitable arrangement that can implement the transmission and reception of network signals, such as wireless network connections, T1 or T3 lines, cable networks, DSL, or telephone lines.

System 1000 can implement any operating system suitable for operating on the network. Software 1050 can be written in any suitable programming language, such as C, C++, Java, or Python. In various examples, application software embodying the functionality of the present disclosure can be deployed in different configurations, such as in a client/server arrangement or through a Web browser as a Web-based application or Web service, for example.

The foregoing description, for the purpose of explanation, has been described with reference to specific examples. However, the illustrative discussions above are not intended to be exhaustive or to limit the invention to the precise forms disclosed. Many modifications and variations are possible in view of the above teachings. The examples were chosen and described in order to best explain the principles of the techniques and their practical applications. Others skilled in the art are thereby enabled to best utilize the techniques and various examples with various modifications as are suited to the particular use contemplated.

Although the disclosure and examples have been fully described with reference to the accompanying figures, it is to be noted that various changes and modifications will become apparent to those skilled in the art. Such changes and modifications are to be understood as being included within the endoscope of the disclosure and examples as defined by the claims. Finally, the entire disclosure of the patents and publications referred to in this application are hereby incorporated herein by reference.

## Claims

1. A system for tracking a location of interest of anatomy of a patient in medical imaging comprising a computing system configured for:
receiving a series of medical imaging video frames captured by a medical imager imaging the anatomy of the patient;
analyzing at least one frame of the series of medical imaging video frames to determine a position of the location of interest relative to the medical imager;
determining at least one estimate of relative motion between the medical imager and the anatomy of the patient; and
tracking the position of the location of interest relative to the medical imager based on the position of the location of interest determined from the at least one frame and the at least one estimate of relative motion between the medical imager and the anatomy of the patient.

2. The system of claim 1, wherein the computing system is configured for receiving medical imager motion data associated with motion of the medical imager and determining the at least one estimate of relative motion between the medical imager and the anatomy of the patient based on the medical imager motion data.

3. The system of claim 2, comprising a motion sensor system configured to be mounted to the medical imager, wherein the medical imager motion data comprises data from the motion sensor system.

4. The system of claim 3, wherein the medical imager is an endoscopic imager and the motion sensor system is mounted to an endoscope of the endoscopic imager, e.g. mounted to a light post of the endoscope.

5. The system of claim 4, wherein the motion sensor system is configured to generate electrical energy from light directed through the light post.

6. The system of any of claims 3-5, wherein the motion sensor system is configured to transmit the data wirelessly.

7. The system of any of claims 3-6, wherein the computing system is configured for determining the at least one estimate of relative motion between the medical imager and the anatomy of the patient based on an offset between a location of the motion sensor system and a predetermined location on the medical imager, wherein the computing system is optionally configured for receiving a user input associated with selection of the offset, and/or determining the offset by imaging an optical calibration object by the medical imager.

8. The system of claim 2, wherein the system comprises at least one sensor that is spaced apart from the medical imager, and wherein the medical imager motion data comprises data from the least one sensor.

9. The system of claim 8, wherein the at least one sensor that is spaced apart from the medical imager comprises a camera that captures images of at least one tracking object associated with the medical imager.

10. The system of any of the preceding claims, wherein the computing system is configured for determining the at least one estimate of relative motion between the medical imager and the anatomy of the patient by analyzing a plurality of frames of the series of medical imaging video frames, e.g. using a machine learning model to identify matched key points in the plurality of frames and determining the at least one estimate of relative motion between the medical imager and the anatomy of the patient based on differences in location of the matched key points, wherein analyzing the plurality of frames of the series of medical imaging video frames optionally comprises determining at least one pixel motion vector.

11. The system of any of the preceding claims, wherein the computing system is configured for analyzing the at least one frame of the series of medical imaging video frames to determine the position of a location of interest by locating the location of interest in the at least one frame based on a position of at least a portion of a tool in the at least one frame, wherein the at least a portion of the tool optionally comprises a tip of the tool and the location of interest is a location of anatomy in the at least one frame that corresponds to the tip of the tool in the at least one frame.

12. The system of any of the preceding claims, wherein the location of interest is a first location of interest, and the computing system is configured for:
determining a position of a second location of interest of the anatomy of the patient relative to the medical imager based on the series of medical imaging video frames; and
determining a distance between the second location of interest and the first location of interest based on a tracked position of the first location of interest relative to the medical imager and the position of the second location of interest relative to the medical imager.

13. The system of any of the preceding claims, wherein the at least one estimate of relative motion between the medical imager and the anatomy of the patient is determined using a first motion estimate at a rate of every M frames, wherein M is greater than one, wherein optionally the at least one estimate of relative motion between the medical imager and the anatomy of the patient is determined using a second motion estimate at a rate of every N frames, wherein N is less than M.

14. The system of any of the preceding claims, wherein the computing system is configured for displaying a visualization comprising a graphical indication of the location of interest in association with the medical imaging video frames, and optionally computing a measurement based on the tracked position of the location of interest and displaying the measurement in the visualization.

15. The system of any of the preceding claims, wherein the computing system is configured for determining the at least one estimate of relative motion between the medical imager and the anatomy of the patient by combining data from different medical imager motion tracking algorithms, wherein optionally the data from the different medical imager motion tracking algorithms is combined using a Kalman filter.

16. A method for tracking a location of interest of anatomy of a patient in medical imaging comprising, at a computing system:
receiving a series of medical imaging video frames captured by a medical imager imaging the anatomy of the patient;
analyzing at least one frame of the series of medical imaging video frames to determine a position of the location of interest relative to the medical imager;
determining at least one estimate of relative motion between the medical imager and the anatomy of the patient; and
tracking the position of the location of interest relative to the medical imager based on the position of the location of interest determined from the at least one frame and the at least one estimate of relative motion between the medical imager and the anatomy of the patient.
